# EUROPEAN PATENT APPLICATION

(11) **EP 4 321 606 A1**
(43) Date of publication of application: **14.02.2024**
(21) Application number: 22189439.7
(22) Date of filing: 09.08.2022
(51) Int. Cl.: C12M 1/00

(54) **DEVICE AND METHOD FOR ILLUMINATING A CHEMICAL AND/OR BIOLOGICAL SAMPLE**

(71) Applicant: Ningaloo Biosystems GmbH, 51105 Köln (DE)
(72) Inventor: Müller-Hartmann, Herbert, 50937 Köln (DE); Schmidt, Hanns-Martin, 50733 Köln (DE)
(74) Representative: Remus, Alvaro Johannes

(57) **Abstract**

The invention relates to a device 1 and related methods for illuminating at least one chemical and/or biological sample 4, comprising at least one internal space 3 which is designed to hold the chemical and/or biological sample 4 or which is designed to comprise at least one container 20 designed to hold at least one chemical and/or biological sample 4, and at least one illumination means 9 for illuminating the chemical and/or biological sample 4 in the internal space 3, wherein at least two light guiding elements 11, 12 comprising means for directing light at least in one direction are disposed between the illumination means 9 and the internal space 3, and wherein at least one first light guiding element 11 comprising means for absorbing light is arranged proximal to the illumination means 9 and at least one second light guiding element 12 comprising means for reflecting light is arranged proximal to the internal space 3.

## Description

### Field of the invention

The invention relates to a device for illuminating at least one chemical and/or biological sample, in particular for photoactivation, photodeactivation or photocatalysis, comprising at least one internal space which is designed to hold the chemical and/or biological sample or which is designed to comprise at least one container designed to hold at least one chemical and/or biological sample, and at least one illumination means for illuminating the chemical and/or biological sample in the internal space. The invention further relates to a method for illuminating at least one chemical and/or biological sample in at least one internal space of a container, or in at least one internal space of said device.

### Background of the invention

Optogenetics is a technology combining the use of light and genetically encoded light-sensitive proteins to control the behavior of living cells and organisms or biochemical processes. Optogenetic technology is increasingly applied in many laboratories around the world and has become a booming field thanks to several technological developments. For example, the search for and combination of functional domains to create novel light-sensitive photoswitches capable of controlling particular cellular behaviors has yielded a diverse set of tools with different functionalities [1,2]. However, although progress in the light-delivery technologies, particularly at the microscopic level, has enabled unprecedented control over *in vitro* and *in vivo* processes through illumination, standardized and useful laboratory or biomanufacturing equipment for employing this promising technology in broader cell-culture based applications is still not available in the market. While to date self-made devices dominate the laboratory landscape, there is an increasing need for technically mature and user-optimized laboratory equipment for employing this promising technology in a reproducible and reliable manner.

Olsen et al. (2014) disclose a method for controlling gene expression dynamics with light using a device that contains independently programmable blue, green, red and far-red light-emitting diodes (LEDs) that deliver calibrated intensities of each wavelength to each of 64 standard test tubes. The device allows optical signals to be sent into exponentially growing bacterial cultures in a shaking incubator. To this end, an array of individually controlled LEDs is disposed underneath the test tubes and used to deliver programmed dynamic light inputs to the bacterial cultures therein, wherein each culture tube is optically isolated with an opaque foam [3].

Gerhardt et al. (2016) disclose a device for delivering two independent light signals to each well of a 24-well culture plate, including a printed circuit board (PCB) outfitted with a Secure Digital (SD) card reader, a microcontroller, 3 LED drivers, 48 solder-free LED sockets, a power regulating circuit, and other standard electronics components. A chassis houses the assembled PCB and 24-well plate. The LEDs are arranged on the PCB which is mounted to a mounting plate disposed underneath the 24-well plate. The black-walled 24-well plate has a transparent plastic bottom and is aligned and held in place by a plate adapter. An adhesive foil plate cover provides a sealed environment for each well. Laser cut nitrile gaskets are placed at each of the interfaces above the PCB to reduce optical contamination. A lid is placed atop the plate and the complete assembly is secured with wing nuts [4].

US 2018/0016538 A1 discloses apparatus, systems and methods relating to an illumination opto-plate configured to specifically illuminate the wells of a cell culture plate. The apparatus has a base and a plate adaptor that are configured to be used in conjunction with a 96-well plate or other common culture plates, such as 6-well, 12-well, 24-well, 48-well, or 384-well plates. The culture plate is placed atop the adaptor such that various openings defined in the adaptor that correspond to LED fixtures are disposed below corresponding wells on the tissue culture plate. The thin profile of the opto-plate allows for use in an incubator or with a microscope.

As the light-sensitive molecules in optogenetic applications are obviously very sensitive to light beams, it is of paramount importance that each sample is treated individually without any interference in order to ensure reproducible and reliable results. Therefore, undesirable exposure of an individual sample to light beams that might, for example, derive from a light source used to illuminate another sample has to be avoided. But, in contrast, it is a drawback of the prior art devices that cross-talk of light beams between neighboring samples cannot be avoided if several samples are treated. In addition, prior art devices provide light to the samples in a way, that the light intensity distribution at the bottom of the individual illuminated samples is according to the directional special light emission characteristics of the illumination means with the light emitting element (LED) employed. The special emission characteristic of an LED light emitting element are typically Lambertian, meaning that at angles less than 90° with respect to the LED surface irradiance is gradually declining, or use lenses to even enhance light emission at the direction close to 90° from the LED surface, thus even showing stronger declines of irradiance at angles less than 90°. This results in the highest intensity area closest to the individual light emitting element, e.g. in the case of 96-well plates at the center of the well bottom. The surrounding areas are illuminated with a lower intensity. As a consequence, exposure of the chemical and/or biological sample occurs in a necessarily inhomogeneous manner, where, for example, different sub-volumes or cells residing in the same sample experience different illumination intensities. This is particularly disadvantageous when no efficient and frequent mixing of the sample is possible that would even out such different illumination intensities, for example in the case of using adherent cells, or low volume internal spaces of the sample container, such as 96-well or 384-well plates, where even mixing of the samples contained even at high horizontal orbital shaking velocities is inefficient, meaning that, for example, the same suspended cells remain either in the central or peripheral areas inside the internal scale/wells. Accordingly, the prior art devices are not designed or equipped for separately illuminating cell cultures or other biological and/or chemical samples in multiwell plates, e.g. 6-well, 12-well, 24-well, 48-well, 96-well, or 384-well plates, in a reproducible and reliable as well as homogeneous manner.

### Summary of the invention

It is an object of the invention to provide a device and related method as initially specified, which allow for effective illumination of individual chemical and/or biological samples in a reproducible and reliable as well as homogeneous manner.

The object is achieved by a device as initially specified, wherein at least two light guiding elements comprising means for directing light at least in one (preferred) direction are disposed between the illumination means and the internal space, and wherein at least one first light guiding element comprising means for absorbing light (in the following also referred to as "Absorbing Tunnel") is arranged proximal to the illumination means and at least one second light guiding element comprising means for reflecting light (in the following also referred to as "Reflecting Tunnel") is arranged proximal to the internal space. That is, such a light guide unit according to the invention is a combination of at least one Absorbing Tunnel and at least one Reflecting Tunnel, wherein the Absorbing Tunnel is arranged close to the illumination means and the Reflecting Tunnel is arranged close to the internal space and thus the biological and/or chemical sample. Benefit of this combination of at least two light guiding elements is to enhance the illumination intensity of an individual sample through the exclusion of light beams with flat inclination angles and the convergence of light beams with steep inclination angles. That is, the beneficial arrangement of the light guiding elements ensures that light beams with flat inclination angles are eliminated by the first light guiding element while light beams with steep inclination angles are directed and focused towards the desired sample by the second light guiding element. In addition, the illumination intensity of an individual sample is enhanced at the periphery, that is, in particular, at the areas farthest from the light emitting element, as compared to not employing the light guiding elements. The device according to the invention thus allows for very effective illumination of individual chemical and/or biological samples in a reproducible and reliable as well as more homogeneous manner.

For example, if several samples are to be treated in multiwell plates, e.g. 6-well, 12-well, 24-well, 48-well, 96-well, or 384-well plates, each individual sample can be treated separately without any interference because undesirable exposure of each individual sample to light beams that derive from a light source used to illuminate another sample ("cross-talk") can be avoided by the device according to the invention. To this end, light beams with flat inclination angles are completely eliminated or reflected backwards by the first light guiding element while only light beams with steep inclination angles can pass through and are directed to the desired sample by the second light guiding element. Thus, the device according to the invention is designed for separately illuminating a plurality of cell cultures or other biological and/or chemical samples in a reproducible and reliable manner.

In addition, as a consequence of the proximity of the second light guiding element to the desired sample and its relative distance from the light emitting element, the direction of light beams with steep inclination angles results in an enhanced illumination intensity of the desired sample close to its periphery, or alternatively the group of samples illuminated by one light guiding element pair positioned at the periphery of the group of samples, which is close to the reflective wall or reflective boundary surface of the second light guiding element. Thus, the previously lower illumination intensity in this area is compensated by the device according to the invention.

For example, the device according to the invention and/or the container may comprise or be at least one of a vessel, a plurality of vessels, and a cell culture vessel or components thereof such as, e.g., a multiwell plate (6-well, 12-well, 24-well, 48-well, 96-well, or 384-well plate etc.).

The first light guiding element ("Absorbing Tunnel") may be, for example, a hollow body such as a tube, a pipe or the like, having a circular or rectangular (square-shaped) cross section and being made of a black and/or opaque material for effectively absorbing light beams with flat inclination angles.

In an advantageous embodiment of the invention, the first light guiding element comprises at least one light absorbing inner wall. For example, the inner wall of the first light guiding element may be coated with a matted paint or velvet coating.

In another advantageous embodiment of the invention, the first light guiding element comprises at least one transparent body having light absorbing properties. The transparent body may have, for example, a square or cylindrical shape, wherein either the four sides of the square perpendicular to the sides for light entry and egress or the curved surface of the cylinder at least partially have light absorbing properties.

In a further advantageous embodiment of the invention, the first light guiding element further comprises at least one reflective surface for reflecting incoming light beams back to the illumination means. Due to this measure, the back reflected light can re-emerge from the illumination means and reach the sample "on a second try", so as to increase the intensity of light and the degree of efficiency of the illumination means. This has the additional advantage that energy of the light beams hitting the walls is not lost but reflected back to its source and can again contribute to the overall output light intensity of the illumination means. Preferably, the first light guiding element comprises four sides or the curved surface of the cylinder equipped with a reflector function.

For example, the second light guiding element may be a "block" (e.g., an upright cylinder) comprising a transparent material having an optical density that is high enough (in relation to the ambient air) to completely reflect (at its lateral sides) the light ingressing through the end wall proximal to the first light guiding element. The material may be, for example, glass or a transparent polymer such as acrylic glass. Such "glass body" works as a kind of circumferential mirror that increases the intensity of the light egressing through the end wall proximal to the internal space.

Accordingly, in an advantageous embodiment of the invention, the second light guiding element comprises at least one transparent cylindric body where light entering through one (end) wall of the body is reflected by the curved boundary surface through total internal reflection, but can egress through the opposite parallel (end) wall.

In an alternative advantageous embodiment of the invention, the second light guiding element comprises at least one cylinder-shaped inner hollow space with a curved mirrored wall and two transparent walls or openings. That is, the second light guiding element may be a body having a curved mirrored wall where light entering through one end wall of the body is reflected by the curved mirror, but can egress through the opposite parallel end wall.

In another advantageous embodiment of the invention, the illumination means comprises at least one light emitting element, wherein one light emitting element is combined with one first light guiding element and/or one second light guiding element. In such embodiment the light emitting element may be positioned on the axis of the light guiding elements in the direction of the light passage (path).

For example, the illumination means may comprise one or more light emitting elements such as light emitting diodes (LEDs).

In another advantageous embodiment of the invention, the illumination means comprises at least two light emitting elements, wherein at least two light emitting elements are combined with one first light guiding element and/or one second light guiding element, wherein the light guiding elements integrate the light for the illumination of one sample. In this embodiment, the light guiding elements can be used, for example, to illuminate a plurality of samples. Benefit of this arrangement is to reduce cross-talk through the exclusion of light beams with flat inclination angles.

In a further advantageous embodiment of the invention, the ratio of the length (l₁) of the first light guiding element and the length (l₂) of the second light guiding element is approximately 1:1. Basically, the ratio of the lengths of the light guiding elements has to be selected such that the intensity of the light hitting the sample, the "steepness" of the light beams ingressing the internal space, and the compensatory balancing of the special distribution of the illumination intensities reaching the sample are optimized.

The object is further achieved by a method as initially specified using at least one illumination means, wherein light is directed at least in one direction from the illumination means to the internal space by means of at least two light guiding elements, and wherein a part of the light is absorbed by at least one first light guiding element arranged proximal to the illumination means and a part of the light is reflected by at least one second light guiding element arranged proximal to the internal space. Due to these measures, the illumination intensity of an individual sample can be significantly enhanced through the exclusion of light beams with flat inclination angles and the convergence of light beams with steep inclination angles. That is, by the method according to the invention light beams with flat inclination angles are eliminated by the first light guiding element while light beams with steep inclination angles are directed and focused towards the desired sample by the second light guiding element. In addition, the illumination intensities of an individual sample, or alternatively the group of samples illuminated by one light guiding element pair positioned at the periphery of the group of samples, are enhanced at the periphery by the method according to the invention, that is, in particular, at the areas farthest from the light emitting element, as compared to not employing the light guiding elements. The method according to the invention thus ensures a very effective illumination of individual chemical and/or biological samples in a reproducible and reliable as well as more homogeneous manner.

In an advantageous embodiment of the invention, the light is directed from the illumination means to the internal space such that at least a majority of the light beams reaching the internal space and/or egressing from the second light guiding element pass along and approximately parallel to the axis of the light guiding elements.

In a further advantageous embodiment of the invention, light beams hitting the inner wall of the first light guiding element are absorbed, preferably completely, so that light beams with flat inclination angles can be effectively eliminated.

In another advantageous embodiment of the invention, incoming light beams are reflected back to the illumination means by at least one reflective surface of the first light guiding element. Due to this measure, the back reflected light can re-emerge from the illumination means and reach the sample "on a second try", so as to increase the intensity of light and the degree of efficiency of the illumination means. This has the additional advantage that energy of the light beams hitting the walls is not lost but reflected back to its source and can again contribute to the overall output light intensity of the illumination means.

In another advantageous embodiment of the invention, light can ingress and egress through two transparent walls or openings of at least one cylinder-shaped inner hollow space of the second light guiding element, and wherein light is reflected by the curved mirrored wall of the cylinder-shaped inner hollow space.

In another advantageous embodiment of the invention, light entering through one wall of at least one transparent cylindric body of the second light guiding element is reflected on the curved boundary layer through total internal reflection, but can egress through the opposite parallel wall.

"Illumination" or "illuminating" as used herein refers to the irradiation of a space, object or sample with light, including but not limited to an active treatment of the space, object or sample with light or the passive exposure of the space, object or sample to light, the latter including a visual inspection or analysis of the space, object or sample.

"Light" as used herein refers to electromagnetic radiation of any wavelength, including both visible and non-visible light, gamma rays, X-rays, microwaves, and radio waves. Visible light has wavelengths in the range of 400-700 nanometers (nm), i.e. between the infrared and the ultraviolet radiation. Infrared (IR) radiation has wavelengths longer than those of visible light, extending from 700 nanometers to 1 millimeter. Ultraviolet (UV) radiation has wavelengths shorter than that of visible light but longer than X-rays, extending from 10 nm to 400 nm.

"Intensity of light" or "light intensity" is used herein as a generic term referring to an undefined (indefinite) quantity or amount of light, including any specific radiometric and photometric value (such as illuminance, irradiance, luminous flux, radiant flux, luminous exposure, and radiant exposure) defining quantities of light.

"Transmittance" or "transparent" as used herein refers to the physical property of allowing the transmission of light through a material, thus non-selectivity regarding wavelengths, acting in the same function as a neutral-density filter. "Transmittance" as used herein includes both "transparency" and "translucency". Transparency is the physical property of allowing light to pass through the material without or almost without scattering of light. Translucency (translucence) is the physical property of allowing light to pass through the material, wherein the light is scattered within or at the surfaces of the material due to a change in the index of refraction.

"Backwards reflection" or "reflected backwards" as used herein refers to a measure where light beams are reflected back to the light source that has emitted these light beams, wherein the outgoing light path angle is identical to the incoming in such a way that the light paths are identical or nearly identical (comparable to a rear reflector), and/or wherein the outgoing light path angle is pi (or 180 degrees) minus the incoming light path angle.

### Brief description of the drawings

**Figure 1** shows a schematic representation (longitudinal section) of an exemplary embodiment of a device according to the invention, wherein one well of a 6-well plate with one "Absorbing Tunnel", one "Reflecting Tunnel", and illumination means are depicted;
**Figure 2** shows a schematic representation (longitudinal section) of another exemplary embodiment of a device according to the invention, wherein four wells of a 16x96-well plate (a cut through a 4x4 block of wells is shown) with one "Absorbing Tunnel", one "Reflecting Tunnel", and illumination means are depicted;
**Figure 3** shows a schematic representation (longitudinal section) of a further exemplary embodiment of a device according to the invention, wherein two wells of a 24-well plate with two "Absorbing Tunnels", two "Reflecting Tunnels", and illumination means are depicted;
**Figure 4** shows a schematic representation (longitudinal section) of a further exemplary embodiment of a device according to the invention, wherein
   a) five wells of a 99-well plate with five "Absorbing Tunnels", five "Reflecting Tunnels" (optical fiber or light guide), as well as illumination means, and
   b) a path of light beams within one of the "Absorbing Tunnels" and one of the "Reflecting Tunnels"
   are depicted;
**Figure 5** shows a schematic representation (longitudinal section) of a further exemplary embodiment of a device according to the invention comprising illumination and ventilation means, wherein
   b) five wells of a 69-well plate with five "Absorbing Tunnels", five "Reflecting Tunnels" (with mirrors), as well as illumination means, and
   b) a path of light beams within one of the "Absorbing Tunnels" and one of the "Reflecting Tunnels"
   are depicted;

### Description of exemplary and preferred embodiments of the invention.

For the sake of clarity, in this description the same/similar components/elements of the different devices shown herein are identified by the same reference numbers, respectively.

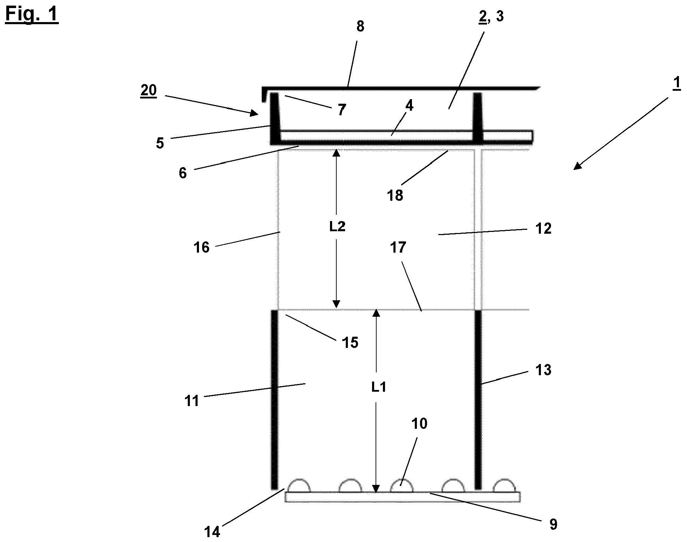

**Figure 1** shows an exemplary embodiment of a device 1 according to the invention. The device 1 is designed to comprise a container 20 comprising six cavities 2 ("wells", of which only one is completely depicted here), each cavity 2 representing an internal space 3 being capable of holding a chemical and/or biological sample 4. The cavities 2 / internal spaces 3 are each partially surrounded by a wall element 5 which is open at the top of each internal space 3. At the bottom of each internal space 3, the wall element 5 comprises windows 6 for illuminating the internal spaces 3. The chemical and/or biological sample(s) 4 can be introduced into the internal space(s) 3 through opening(s) 7. The openings 7 are covered by a lid 8 extending to all cavities 2. In order to protect the internal spaces 3 from the ingress of light as well as prevent "cross-talk" between individual cavities 2 / internal spaces 3 when illuminated separately, the wall elements 5 and the lid 8 each comprise a light-proof material. Each window 6 is either permanently transparent or can be switched between a first state ("closed state") and one or more second state(s) ("active state") by means of a switchable element (not shown). In the closed state light is blocked off from the internal space 3, while in the active state light can pass through the window 6 into and out of the internal space 3 in a controlled manner.

Illumination of the internal spaces 3 is employed by means of an illumination means 9 comprising a multitude of light emitting elements 10 (e.g., LEDs). When the light emitting elements 10 are active, the light emitted by them passes through light guiding elements 11, 12, two of which being disposed underneath each cavity 2 between the illumination means 9 and the internal space 3. The light guiding elements 11, 12 direct the light from the illumination means 9 in the direction of the internal space 3. The first light guiding element 11 ("Absorbing Tunnel") is arranged proximal to the illumination means 9 and comprises means for absorbing light. In particular, the first light guiding element 11 is a hollow body (such as a tube having a circular cross section), wherein the wall 13 of this body is made of a black and/or opaque material for effectively absorbing light beams with flat inclination angles. Alternatively, the inner surface of the wall 13 of the first light guiding element 11 can be coated with a matted paint or velvet coating or equipped with a coating or structured in a way to ensure backwards reflection. The first light guiding element further comprises two openings 14, 15, one opening 14 proximal to the illumination means 9 and one opening 15 proximal to the second light guiding element 12. The light emitted from the light emitting elements 10 ingresses through the opening 14 into the hollow body and egresses the body at the opening 15, wherein light beams with flat inclination angles are absorbed or reflected backwards by the wall 13 and light beams with steep inclination angles pass through the hollow body. The second light guiding element 12 ("Reflecting Tunnel") is arranged proximal to the internal space 3 (and thus the biological and/or chemical sample 4) and comprises means for reflecting light. In particular, the second light guiding element 12 is a solid "block" having a circular cross section (upright cylinder) and comprises a transparent material (e.g., glass or acrylic glass) having an optical density that is high enough (in relation to the ambient air) to completely reflect (at its lateral sides 16) the light ingressing through the end wall 17 proximal to the first light guiding element 11. The second light guiding element 12 works as a kind of circumferential mirror that increases the intensity of the light egressing through the end wall 18 proximal to the internal space 3.

The diameters of the circular cross sections of both light guiding elements 11, 12 are adapted to the diameter of the cross-section of the circular cavity 2 in order to fit the dimensions of the multiwell plate used (here container 20). Moreover, the ratio of the length (L1) of the first light guiding element 11 and the length (L2) of the second light guiding element 12 is approximately 1:1 so that both the intensity of the light hitting the sample 4 and the "steepness" of the light beams ingressing the internal space 3 are balanced.

The illumination means 9 comprises several light emitting elements 10, wherein sixteen or twelve light emitting elements 10 (only four shown in the cross-section view) are combined with the first light guiding element 11 and the second light guiding element 12. In this embodiment, the light guiding elements 11, 12 integrate the light of the four light emitting elements 10 for the illumination of one sample 4.

The combination of the light guiding elements 11, 12 allows for enhancing the illumination intensity of each individual sample 4, wherein light beams with flat inclination angles are eliminated by the first light guiding element 11 and light beams with steep inclination angles are directed and focused towards the desired sample 4 by the second light guiding element 12. The device 1 thus allows for very effective illumination (e.g., optogenetic control, photoactivation, photodeactivation and/or photocatalysis) of individual chemical and/or biological samples 4 in a reproducible and reliable as well as more homogeneous manner.

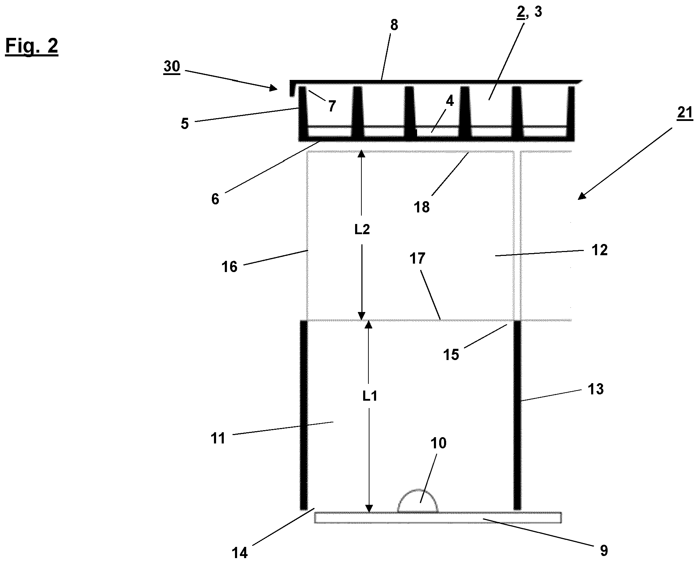

**Figure 2** shows another exemplary embodiment of a device 21 according to the invention. The device 21 is designed to comprise a container 30 comprising a plurality of cavities 2 ("wells", of which only five are depicted here), each cavity 2 representing an internal space 3 being capable of holding a chemical and/or biological sample 4. The cavities 2 / internal spaces 3 are each partially surrounded by a wall element 5 which is open at the top of each internal space 3. At the bottom of each internal space 3, the wall element 5 comprises windows 6 for illuminating the internal spaces 3. The chemical and/or biological sample(s) 4 can be introduced into the internal spaces 3 through openings 7. The openings 7 are covered by a lid 8 extending to all cavities 2. In order to protect the internal spaces 3 from the ingress of light as well as prevent "cross-talk" between individual cavities 2 / internal spaces 3 when illuminated through independent pairs of light guiding elements 11, 12, the wall elements 5 and the lid 8 each comprise a light-proof material. Each window 6 is either permanently transparent or can be switched between a first state ("closed state") and one or more second state(s) ("active state") by means of a switchable element (not shown). In the closed state light is blocked off from the internal space 3, while in the active state light can pass through the window 6 into and out of the internal space 3 in a controlled manner.

Illumination of the internal spaces 3 is employed by means of an illumination means 9 comprising a multitude of light emitting elements 10 (e.g., LEDs), one of which is shown here. When the light emitting elements 10 are active, the light emitted by them passes through light guiding elements 11, 12, two of which being disposed underneath a group of cavities 2 between the illumination means 9 and the internal spaces 3. The light guiding elements 11, 12 direct the light from the illumination means 9 In the direction of the internal spaces 3. The first light guiding element 11 ("Absorbing Tunnel") is arranged proximal to the illumination means 9 and comprises means for absorbing light. In particular, the first light guiding element 11 is a hollow body (such as a tube) having a circular cross section, wherein the wall 13 of this body is made of a black and/or opaque material for effectively absorbing light beams with flat inclination angles. Alternatively, the inner surface of the wall 13 of the first light guiding element 11 can be coated with a matted paint or velvet coating or equipped with a coating or structured in a way to ensure backwards reflection where the outgoing light path angle is identical to the incoming in such a way that the light paths are identical or nearly identical (comparable to a rear reflector). The first light guiding element further comprises two openings 14, 15, one opening 14 proximal to the illumination means 9 and one opening 15 proximal to the second light guiding element 12. The light emitted from the light emitting elements 10 ingresses through the opening 14 into the hollow body and egresses the body at the opening 15, wherein light beams with flat inclination angles are absorbed or reflected backwards by the wall 13 and light beams with steep inclination angles pass through the hollow body. The second light guiding element 12 ("Reflecting Tunnel") is arranged proximal to the internal space 3 (and thus the biological and/or chemical sample 4) and comprises means for reflecting light. In particular, the second light guiding element 12 is a solid "block" having a circular cross section (upright cylinder) and comprises a transparent material (e.g., glass or acrylic glass) having an optical density that is high enough (in relation to the ambient air) to completely reflect (at its lateral sides 16) the light ingressing through the end wall 17 proximal to the first light guiding element 11. The second light guiding element 12 works as a kind of circumferential mirror that increases the intensity of the light egressing through the end wall 18 proximal to the internal space 3.

The ratio of the length (L1) of the first light guiding element 11 and the length (L2) of the second light guiding element 12 is approximately 1:1 so that both the intensity of the light hitting the sample 4 and the "steepness" of the light beams ingressing the internal space 3 are balanced.

The illumination means 9 comprises several light emitting elements 10, wherein only one light emitting element 10 is combined with the first light guiding element 11 and the second light guiding element 12. In this embodiment, the light guiding elements 11, 12 integrate the light for the illumination of a group of four samples 4 so that the illumination intensities of these sixteen different samples 4 (only four shown in the cross-section view) positioned next to each other are equilibrated (samples or wells central vs marginal positions with regard to the illumination means).

The combination of the light guiding elements 11, 12 allows for enhancing the illumination intensity of each individual sample 4, wherein light beams with flat inclination angles are eliminated by the first light guiding element 11 and light beams with steep inclination angles are directed and focused towards the desired sample 4 by the second light guiding element 12. In addition, the illumination intensity of an individual sample 4 positioned at the periphery of an individual pair of light guiding elements 11, 12 is enhanced, i.e., at the areas farthest from the light emitting element 10, as compared to not employing the light guiding elements 11, 12. As a consequence of the proximity of the second light guiding element 12 to the desired samples 4 and its relative distance from the light emitting element 10, the direction of light beams with steep inclination angles further results in an enhanced illumination intensity of all desired samples 4 close to the periphery of the group of samples 4 illuminated by one pair of light guiding elements 11, 12, which is close to the reflective wall or reflective boundary surface of the second light guiding element 12. Thus, the previously lower illumination intensity in this area is compensated by the device according to the invention. The device 1 thus allows for very effective illumination (e.g., optogenetic control, photoactivation, photodeactivation and/or photocatalysis) of individual chemical and/or biological samples 4 in a reproducible and reliable as well as more homogeneous (less dependent from their positioning) manner.

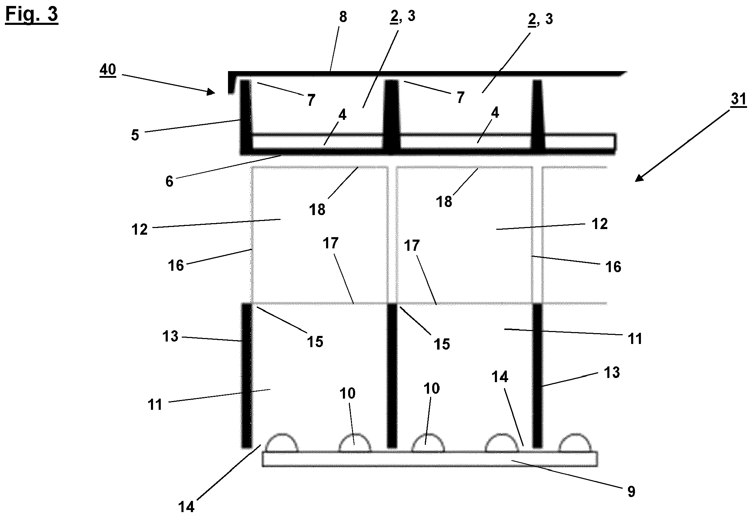

**Figure 3** shows a further exemplary embodiment of a device 31 according to the invention. The device 31 is designed to comprise a container 40 comprising twenty-four cavities 2 ("wells", of which two are completely depicted here), each cavity 2 representing an internal space 3 being capable of holding a chemical and/or biological sample 4. The cavities 2 / internal spaces 3 are each partially surrounded by a wall element 5 which is open at the top of each internal space

3. At the bottom of each internal space 3, the wall element 5 comprises windows 6 for illuminating the internal spaces 3. The chemical and/or biological sample(s) 4 can be introduced into the internal spaces 3 through openings 7. The openings 7 are covered by a lid 8 extending to all cavities 2. In order to protect the internal spaces 3 from the ingress of light as well as prevent "cross-talk" between individual cavities 2 / internal spaces 3 illuminated through independent pairs of light guiding elements 11, 12, the wall elements 5 and the lid 8 each comprise a light-proof material. Each window 6 is either permanently transparent or can be switched between a first state ("closed state") and one or more second state(s) ("active state") by means of a switchable element (not shown). In the closed state light is blocked off from the internal space 3, while in the active state light can pass through the window 6 into and out of the internal space 3 in a controlled manner.

Illumination of the internal spaces 3 is employed by means of an illumination means 9 comprising a multitude of light emitting elements 10 (e.g., LEDs). When the light emitting elements 10 are active, the light emitted by them passes through light guiding elements 11, 12, two of which being disposed underneath each cavity 2 between the illumination means 9 and the internal space 3. The light guiding elements 11, 12 direct the light from the illumination means 9 in the direction of the internal space 3. The first light guiding element 11 ("Absorbing Tunnel") is arranged proximal to the illumination means 9 and comprises means for absorbing light. In particular, the first light guiding element 11 is a hollow body (such as a tube) having a circular cross section, wherein the wall 13 of this body is made of a black and/or opaque material for effectively absorbing light beams with flat inclination angles. Alternatively, the inner surface of the wall 13 of the first light guiding element 11 can be coated with a matted paint or velvet coating or equipped with a coating or structured in a way to ensure backwards reflection where the outgoing light path angle is identical to the incoming in such a way that the light paths are identical or nearly identical (comparable to a rear reflector). The first light guiding element further comprises two openings 14, 15, one opening 14 proximal to the illumination means 9 and one opening 15 proximal to the second light guiding element 12. The light emitted from the light emitting elements 10 ingresses through the opening 14 into the hollow body and egresses the body at the opening 15, wherein light beams with flat inclination angles are absorbed or reflected backwards by the wall 13 and light beams with steep inclination angles pass through the hollow body. The second light guiding element 12 ("Reflecting Tunnel") is arranged proximal to the internal space 3 (and thus the biological and/or chemical sample 4) and comprises means for reflecting light. In particular, the second light guiding element 12 is a solid "block" having a circular cross section (upright cylinder) and comprises a transparent material (e.g., glass or acrylic glass) having an optical density that is high enough (in relation to the ambient air) to completely reflect (at its lateral sides 16) the light ingressing through the end wall 17 proximal to the first light guiding element 11. The second light guiding element 12 works as a kind of circumferential mirror that increases the intensity of the light egressing through the end wall 18 proximal to the internal space 3.

The diameters of the circular cross sections of all light guiding elements 11, 12 are adapted to the diameter of the cross section of the circular cavities 2 in order to fit the dimensions of the multiwell plate used (here container 40).

The illumination means 9 comprises several light emitting elements 10, wherein four light emitting elements 10 (only two shown in the cress-section view) are combined with one first light guiding element 11 and one second light guiding element 12, respectively. In this embodiment, one combination of light guiding elements 11, 12 integrate the light of the four light emitting elements 10 for the illumination of one sample 4. That is, if several samples 4 are to be treated in a multiwell plate (here 24-wells), each individual sample 4 can be treated separately without any interference because undesirable exposure of each individual sample 4 to light beams that derive from the four light emitting elements 10 used to illuminate another sample 4 ("cross-talk") can be avoided. To this end, light beams with flat inclination angles are completely eliminated or reflected backwards by the first light guiding elements 11 while only light beams with steep inclination angles can pass through and are directed to the desired sample 4 by the second light guiding element 12. Thus, the device 31 is designed for separately illuminating a plurality of biological and/or chemical samples 4 of the container 40 in a reproducible and reliable manner.

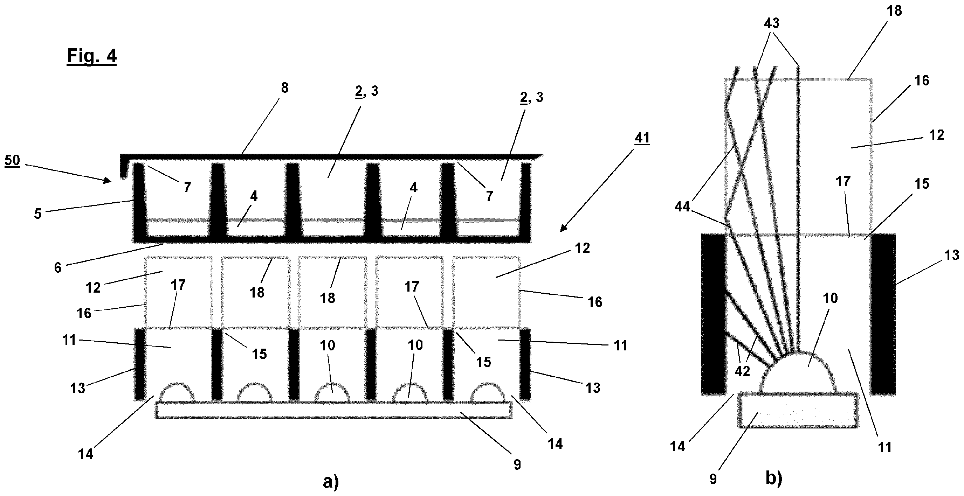

**Figure 4a** shows a further exemplary embodiment of a device 41 according to the invention. The device 41 is designed to comprises a container 50 comprising ninety-six cavities 2 ("wells", of which five are depicted here), each cavity 2 representing an internal space 3 being capable of holding a chemical and/or biological sample 4. The cavities 2 / internal spaces 3 are each partially surrounded by a wall element 5 which is open at the top of each internal space 3. At the bottom of each internal space 3, the wall element 5 comprises windows 6 for illuminating the internal spaces 3. The chemical and/or biological sample(s) 4 can be introduced into the internal spaces 3 through openings 7. The openings 7 are covered by a lid 8 extending to all cavities 2. In order to protect the internal spaces 3 from the ingress of light as well as prevent "cross-talk" between individual cavities 2 / internal spaces 3 illuminated through independent pairs of light guiding elements 11, 12, the wall elements 5 and the lid 8 each comprise a light-proof material. Each window 6 is either permanently transparent or can be switched between a first state ("closed state") and one or more second state(s) ("active state") by means of a switchable element (not shown). In the closed state light is blocked off from the internal space 3, while in the active state light can pass through the window 6 into and out of the internal space 3 in a controlled manner.

Illumination of the internal spaces 3 is employed by means of an illumination means 9 comprising a multitude of light emitting elements 10 (e.g., LEDs). When the light emitting elements 10 are active, the light emitted by them passes through light guiding elements 11, 12, two of which being disposed underneath each cavity 2 between one of the light emitting elements 10 and the internal space 3. The light guiding elements 11, 12 direct the light from the light emitting element 10 in the direction of the internal space 3 aligned therewith. The first light guiding element 11 ("Absorbing Tunnel") is arranged proximal to the illumination means 9 and comprises means for absorbing light. In particular, the first light guiding element 11 is a hollow body (such as a tube) having a circular cross section, wherein the wall 13 of this body is made of a black and/or opaque material for effectively absorbing light beams with flat inclination angles. Alternatively, the inner surface of the wall 13 of the first light guiding element 11 can be coated with a matted paint or velvet coating or equipped with a coating or structured in a way to ensure backwards reflection where the outgoing light path angle is identical to the incoming in such a way that the light paths are identical or nearly identical (comparable to a rear reflector). The first light guiding element further comprises two openings 14, 15, one opening 14 proximal to the illumination means 9 and one opening 15 proximal to the second light guiding element 12. The light emitted from the light emitting elements 10 ingresses through the opening 14 into the hollow body and egresses the body at the opening 15, wherein light beams with flat inclination angles 42 are absorbed or reflected backwards by the wall 13 and light beams with steep inclination angles 43 pass through the hollow body **(****Figure 4b****).** The second light guiding element 12 ("Reflecting Tunnel") is arranged proximal to the internal space 3 (and thus the biological and/or chemical sample 4) and comprises means for reflecting light. In particular, the second light guiding element 12 is a solid "block" having a circular cross section (upright cylinder) and comprises a transparent material (e.g., glass or acrylic glass) having an optical density that is high enough (in relation to the ambient air) to completely reflect (at its lateral sides 16) the light beams 44 ingressing through the end wall 17 proximal to the first light guiding element 11 **(****Figure 4b****).** The second light guiding element 12 works as a kind of circumferential mirror that increases the intensity of the light egressing through the end wall 18 proximal to the internal space 3.

The diameters of the circular cross sections of all light guiding elements 11, 12 are adapted to the diameter of the cross section of the circular cavities 2 in order to fit the dimensions of the multiwell plate used (here container 50).

The illumination means 9 comprises several light emitting elements 10, wherein only one light emitting element 10 is combined with one first light guiding element 11 and one second light guiding element 12, respectively. In this embodiment, each combination of light guiding elements 11, 12 directs the light of one light emitting element 10 to one internal space 3 (cavity 2) of the container 50. That is, if several samples 4 are to be treated in a multiwell plate (here 96-wells), each individual sample 4 can be treated separately without any interference because undesirable exposure of each individual sample 4 to light beams that derive from a light emitting element 10 used to illuminate another sample 4 ("cross-talk") can be avoided. To this end, light beams with flat inclination angles 42 are completely eliminated or reflected backwards by the first light guiding elements 11 while only light beams with steep inclination angles 43 can pass through and are directed to the desired sample 4 by the second light guiding element 12. In addition, the illumination intensity of an individual sample 4 is enhanced at the periphery, i.e., at the areas farthest from the corresponding light emitting element 10, as compared to not employing the light guiding elements 11, 12. As a consequence of the proximity of the second light guiding elements 12 to the desired samples 4 and its relative distance from the light emitting element 10, the direction of light beams with steep inclination angles further results in an enhanced illumination intensity of all desired samples 4 close to their periphery, which is close to the reflective wall or reflective boundary surface of the second light guiding element 12, through the light beams 44 reflected by the second light guiding elements 12. Thus, the previously lower illumination intensity in this area is compensated by the device according to the invention. Thus, the device 41 is designed for separately illuminating a plurality of biological and/or chemical samples 4 of the container 50 in a reproducible and reliable as well as more homogeneous manner.

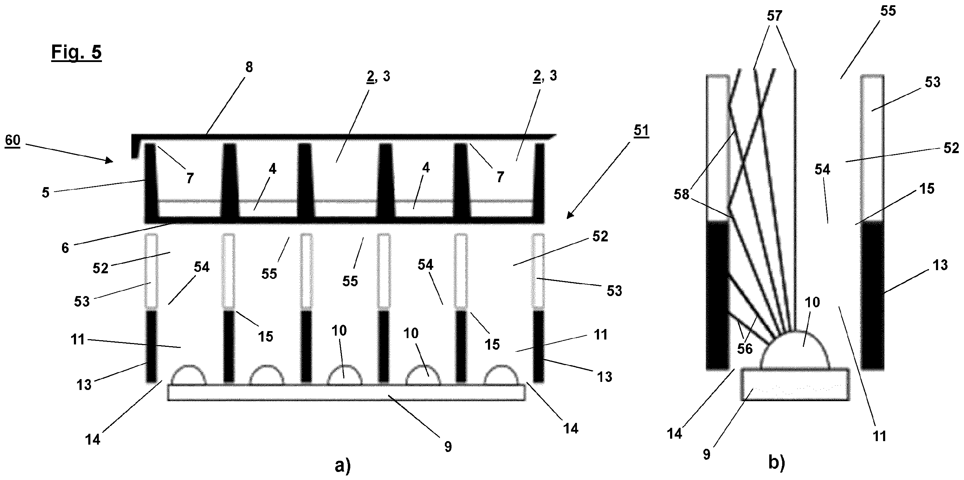

**Figure 5a** shows a further exemplary embodiment of a device 51 according to the invention. The device 51 is designed to comprises a container 60 comprising ninety-six cavities 2 ("wells", of which five are depicted here), each cavity 2 representing an internal space 3 being capable of holding a chemical and/or biological sample 4. The cavities 2 / internal spaces 3 are each partially surrounded by a wall element 5 which is open at the top of each internal space 3. At the bottom of each internal space 3, the wall element 5 comprises windows 6 for illuminating the internal spaces 3. The chemical and/or biological sample(s) 4 can be introduced into the internal spaces 3 through openings 7. The openings 7 are covered by a lid 8 extending to all cavities 2. In order to protect the internal spaces 3 from the ingress of light as well as prevent "cross-talk" between individual cavities 2 / internal spaces 3 illuminated through independent pairs of light guiding elements 11, 12, the wall elements 5 and the lid 8 each comprise a light-proof material. Each window 6 is either permanently transparent or can be switched between a first state ("closed state") and one or more second state(s) ("active state") by means of a switchable element (not shown). In the closed state light is blocked off from the internal space 3, while in the active state light can pass through the window 6 into and out of the internal space 3 in a controlled manner.

Illumination of the internal spaces 3 is employed by means of an illumination means 9 comprising a multitude of light emitting elements 10 (e.g., LEDs). When the light emitting elements 10 are active, the light emitted by them passes through light guiding elements 11, 52, two of which being disposed underneath each cavity 2 between one of the light emitting elements 10 and the internal space 3. The light guiding elements 11, 52 direct the light from the light emitting element 10 in the direction of the internal space 3 aligned therewith. The first light guiding element 11 ("Absorbing Tunnel") is arranged proximal to the illumination means 9 and comprises means for absorbing light. In particular, the first light guiding element 11 is a hollow body (such as a tube) having a circular cross section, wherein the wall 13 of this body is made of a black and/or opaque material for effectively absorbing light beams with flat inclination angles. Alternatively, the inner surface of the wall 13 of the first light guiding element 11 can be coated with a matted paint or velvet coating or equipped with a coating or structured in a way to ensure backwards reflection where the outgoing light path angle is identical to the incoming in such a way that the light paths are identical or nearly identical (comparable to a rear reflector). The first light guiding element further comprises two openings 14, 15, one opening 14 proximal to the illumination means 9 and one opening 15 proximal to the second light guiding element 12. The light emitted from the light emitting elements 10 ingresses through the opening 14 into the hollow body and egresses the body at the opening 15, wherein light beams with flat inclination angles 56 are absorbed or reflected backwards by the wall 13 and light beams with steep inclination angles 57, 58 pass through the hollow body **(****Figure 5b****).** The second light guiding element 52 ("Reflecting Tunnel") is arranged proximal to the internal space 3 (and thus the biological and/or chemical sample 4) and comprises means for reflecting light. In this embodiment, the second light guiding element 52 is a cylinder-shaped hollow body (such as a tube) with a curved mirrored wall 53 and two openings 54, 55, wherein the wall 53 of this body is a curved mirror for completely reflecting the light beams 58 ingressing through the opening 54 proximal to the first light guiding element 11. That is, the second light guiding element 52 is a hollow body having a curved mirrored wall 53 where light beams 58 entering through one opening 54 of the body and having a flat inclination angle are reflected by the curved mirror, and where the light beams having a very flat inclination angle 57 can pass through the hollow body without reflection, but can egress through the opposite opening 55 proximate to the internal space 3 **(****Figure 5b****).**

The diameters of the circular cross sections of all light guiding elements 11, 52 are adapted to the diameter of the cross section of the circular cavities 2 in order to fit the dimensions of the multiwell plate used (here container 60).

The illumination means 9 comprises several light emitting elements 10, wherein only one light emitting element 10 is combined with one first light guiding element 11 and one second light guiding element 52, respectively. In this embodiment, each combination of light guiding elements 11, 52 directs the light of one light emitting element 10 to one internal space 3 (cavity 2) of the container 60. That is, if several samples 4 are to be treated in a multiwell plate (here 96-wells), each individual sample 4 can be treated separately without any interference because undesirable exposure of each individual sample 4 to light beams that derive from a light emitting element 10 used to illuminate another sample 4 ("cross-talk") can be avoided. To this end, light beams with flat inclination angles 56 are completely eliminated or reflected backwards by the first light guiding elements 11 while only light beams with steep inclination angles 57 can pass through and are directed to the desired sample 4 by the second light guiding element 52. In addition, the illumination intensity of an individual sample 4 is enhanced at the periphery, i.e., at the areas farthest from the corresponding light emitting element 10, as compared to not employing the light guiding elements 11, 52. As a consequence of the proximity of the second light guiding elements 52 to the desired samples 4 and its relative distance from the light emitting element 10, the direction of light beams with steep inclination angles further results in an enhanced illumination intensity of all desired samples 4 close to their periphery, which is close to the reflective wall or reflective boundary surface of the second light guiding element 52, through the light beams 58 reflected by the second light guiding elements 52. Thus, the previously lower illumination intensity in this area is compensated by the device according to the invention. Thus, the device 51 is designed for separately illuminating a plurality of biological and/or chemical samples 4 of the container 60 in a reproducible and reliable as well as more homogeneous manner.

### References:

1. Anonym: Method of the Year 2010, Nature Methods VOL. 8 NO. 1, JANUARY 2011, 1-1, published online 20 December 2010, doi:10.1038/nmeth.f.321.
2. Nicole A. Repina, Alyssa Rosenbloom, Abhirup Mukherjee, David V. Schaffer & Ravi S. Kane: AT LIGHT SPEED: Advances in Optogenetic Systems for Regulating Cell Signaling and Behavior, Annu Rev Chem Biomol Eng. 2017 June 07; 8: 13-39. doi:10.1146/annurevchembioeng-060816-101254
3. Evan J. Olson, Lucas A. Hartsough, Brian P. Landry, Raghav Shroff & Jeffrey J. Tabor: Characterizing bacterial gene circuit dynamics with optically programmed gene expression signals, Nature Methods VOL.11 NO.4, APRIL 2014, 449.
4. Karl P. Gerhardt, Evan J. Olson, Sebastian M. Castillo-Hair, Lucas A. Hartsough, Brian P. Landry, Felix Ekness, Rayka Yokoo, Eric J. Gomez, Prabha Ramakrishnan, Junghae Suh, David F. Savage & Jeffrey J. Tabor: An open-hardware platform for optogenetics and photobiology, Scientific Reports 6:35363, DOI: 10.1038/srep35363.

## Claims

1. Device (1, 21, 31, 41, 51) for illuminating at least one chemical and/or biological sample (4), comprising at least one internal space (3) which is designed to hold the chemical and/or biological sample (4) or which is designed to comprise at least one container (20, 30, 40, 50, 60) designed to hold at least one chemical and/or biological sample (4), and at least one illumination means (9) for illuminating the chemical and/or biological sample (4) in the internal space (3), **characterized in that** at least two light guiding elements (11, 12, 52) comprising means for directing light at least in one direction are disposed between the illumination means (9) and the internal space (3), wherein at least one first light guiding element (11) comprising means for absorbing light is arranged proximal to the illumination means (9) and at least one second light guiding element (12, 52) comprising means for reflecting light is arranged proximal to the internal space (3).

2. Device according to claim 1, **characterized in that** the first light guiding element (11) comprises at least one light absorbing inner wall.

3. Device according to claim 1 or 2, **characterized in that** the first light guiding element (11) comprises at least one transparent body having light absorbing properties.

4. Device according to any one of claims 1 to 3, **characterized in that** the first light guiding element (11) further comprises at least one reflective surface for reflecting incoming light beams back to the illumination means (9).

5. Device according to any one of claims 1 to 4, **characterized in that** the second light guiding element (12) comprises at least one transparent cylindric body where light entering through one wall (17) of the body is reflected by the curved boundary surface through total internal reflection, but can egress through the opposite parallel wall (18).

6. Device according to any one of claims 1 to 5, **characterized in that** the second light guiding element (52) comprises at least one cylinder-shaped inner hollow space with a curved mirrored wall (53) and two transparent walls or openings (54, 55).

7. Device according to any one of claims 1 to 6, **characterized in that** the illumination means (9) comprises at least one light emitting element (10), wherein one light emitting element (10) is combined with one first light guiding element (11) and/or one second light guiding element (12, 52).

8. Device according to any one of claims 1 to 7, **characterized in that** the illumination means (9) comprises at least two light emitting elements (10), wherein at least two light emitting elements (10) are combined with one first light guiding element (11) and/or one second light guiding element (12, 52), wherein the light guiding elements (10) integrate the light for the illumination of one sample (4).

9. Device according to any one of claims 1 to 8, **characterized in that** the ratio of the length (L1) of the first light guiding element (11) and the length (L2) of the second light guiding element (12, 52) is approximately 1:1.

10. Method for illuminating at least one chemical and/or biological sample in at least one internal space of a container, or in at least one internal space of the device according to any of claims 1 to 9, using at least one illumination means, wherein light is directed at least in one direction from the illumination means to the internal space by means of at least two light guiding elements, and wherein a part of the light is absorbed by at least one first light guiding element arranged proximal to the illumination means and a part of the light is reflected by at least one second light guiding element arranged proximal to the internal space.

11. Method according to claim 10, wherein the light is directed from the illumination means to the internal space such that at least a majority of the light beams reaching the internal space and/or egressing from the second light guiding element pass along and approximately parallel to the axis of the light guiding elements.

12. Method according to claim 10 or 11, wherein light beams hitting the inner wall of the first light guiding element are absorbed.

13. Method according to any one of claims 10 to 12, wherein incoming light beams are reflected back to the illumination means by at least one reflective surface of the first light guiding element.

14. Method according to any one of claims 10 to 13, wherein light can ingress and egress through two transparent walls or openings of at least one cylinder-shaped inner hollow space of the second light guiding element, and wherein light is reflected by the curved mirrored wall of the cylinder-shaped inner hollow space.

15. Method according to any one of claims 10 to 14, wherein light entering through one wall of at least one transparent cylindric body of the second light guiding element is reflected on the curved boundary layer through total internal reflection, but can egress through the opposite parallel wall.
